# EUROPEAN PATENT APPLICATION

(11) **EP 2 517 733 A1**
(43) Date of publication of application: **31.10.2012**
(21) Application number: 10839646.6
(22) Date of filing: 15.06.2010
(51) Int. Cl.: A61L 2/06, A61L 2/04

(54) **STEAM DISINFECTION APPARATUS HAVING HEATING AMPLIFICATION MEANS**

(30) Priority: 24.12.2009 KR 20090130878
(71) Applicant: Aqua Doctor Co., Ltd., Saha-gu, Busan 604-040 (KR)
(72) Inventor: CHEONG, Yeon-Seong, Busan 602-750 (KR); SON, Soo-Beom, Busan 602-091 (KR)
(74) Representative: Gassner, Wolfgang
(86) International application number: PCT/KR2010/003847
(87) International publication number: WO 2011/078449

(57) **Abstract**

According to the present invention, the steam generated by a steam generator is reheated by a heating amplifying means while being injected through an injection gun, so a temperature of the steam can be increased to a desired range, and accordingly the sterilizing and cleaning effects can be further improved.

## Description

### TECHNICAL FIELD

The present invention relates to an apparatus for sterilization by steam, which generates and injects steam, and more particularly to a steam sterilization apparatus that may increase a temperature of steam to a desired range by reheating the steam, which is generated by a steam generator and injected through an injection gun, by means of a heating amplifying means and accordingly further improve sterilization and cleaning effects.

### BACKGROUND ART

A steam sterilization apparatus is used for instantly heating and evaporating water to generate a high-temperature steam and then injecting the steam, and the stem sterilization apparatus is widely used for cleaning flow tubes or interior of steam cleaners and steam car washers as well as chilling/heating machines, beverage vending machines and air conditioners.

The steam sterilization apparatus generally includes a steam generator for heating water to generate steam, and an injection gun for intermittently injecting the generated steam toward a target.

The steam generator is generally connected to the injection gun by means of a tube with a length of about 1 to 2 m, so the steam generated by the steam generator is naturally cooled while being transferred to the injection gun through the tube. In this reason, in many cases, the steam injected to the outside frequently has a lower temperature than a desired level.

For example, a steam generated by the steam generator generally has a temperature of about 130°C, but a steam injected to the outside through the injection gun via the tube generally has a temperature of about 90°C.

If the injected steam has a lowered temperature, sterilization/disinfection effects for removing molds or bacteria and cleaning/washing functions are deteriorated.

### DISCLOSURE

### Technical Problem

The present invention is designed to solve the problems of the prior art, and therefore it is an object of the present invention to provide an apparatus for sterilization by steam, which may reheat a steam, generated by a steam generator and transferred via a tube, to a desired temperature by means of a heating amplifying means before the steam is finally injected, thereby giving a steam of a suitable temperature in accordance with its usage and thus improving sterilization, disinfection and cleaning effects.

### Technical Solution

In order to accomplish the above object, the present invention provides an apparatus for sterilization by steam, which includes a steam generator for heating water to generate a steam; an injection gun for injecting the steam generated by the steam generator; a flow tube acting as a path through which the steam generated by the steam generator moves to the injection gun; and a heating amplifying means installed at the injection gun to reheat the steam, moved through the flow tube, to a predetermined temperature before being injected.

Preferably, the heating amplifying means includes an amplification tube through which the steam moved via the flow tube is moved; a heating means for heating the amplification tube; and an insulation layer for intercepting a heat of the heating means, which is transferred to the outside.

As an alternative, the heating amplifying means according to the present invention may include a heater with a rod shape; a steam moving tube wound around the heater so as to be capable of being heated by the heater, the steam moving tube allowing the steam to move therein; and an insulation layer installed to an outer side of the wound steam moving tube. The steam moved via the flow tube is heated by the heater while the steam is moving through the steam moving tube.

As an alternative, the heating amplifying means may include a heating coil tube installed in connection with the flow tube and allowing the steam to be movable therein, the heating coil tube being wound into a coil shape and capable of being heated by a power source; and an insulation layer installed to an outer side of the wound heating coil tube. The steam moved via the flow tube is heated while the steam is moving through the heating coil tube.

As an alternative, the heating amplifying means may include an amplification tube through which the steam moved via the flow tube moves; a heating coil installed in the amplification tube and capable of being heated by a power source, the heating coil being wound into a coil shape; a delaying means installed in the amplification tube to delay a moving speed of the steam that is moving through the amplification tube; and an insulation layer installed around the amplification tube. The steam moving through the amplification tube is heated by the heating coil.

As an alternative, the heating amplifying means may include an amplification tube through the steam moved via the flow tube moves; a heater installed in the amplification tube and capable of being heated by a power source, the heater having a rod shape; a coil wound around the heater along a length direction of the heater in the amplification tube; and an insulation layer installed around the amplification tube. The steam moving through the amplification tube is heated by the heater.

Preferably, the heater and the coil are installed at a predetermined interval such that the steam is capable of moving between the heater and the coil, and the amplification tube and the coil are installed at a predetermined interval such that the steam is capable of moving between the heating amplification tube and the coil.

More preferably, the apparatus for sterilization by steam further includes a delaying means for decreasing a flow rate of the steam moving in the amplification tube.

As an example of the delaying means, a spring inserted into the amplification tube may be used.

Another example of the delaying means, a plurality of interference plates alternately formed at different locations of an inner side of the amplification tube may be used.

Preferably, the amplification tube has a bent shape to form a plurality of passages.

In addition, the amplification tube may further include a heat plate additionally installed to a part of an inner side of the amplification tube.

Moreover, a coupler for attaching or detaching a cleaning nozzle may be installed at an outlet of the heating amplifying means.

Here, the apparatus for sterilization by steam preferably has a heat sink, wherein the heat sink is provided at an outside of the insulation layer and has a plurality of circulation holes formed therein and a spacer protruded on an inner side thereof.

### DESCRIPTION OF DRAWINGS

Fig. 1 is a schematic view schematically showing an apparatus for sterilization by steam (hereinafter, referred to as an 'steam sterilization apparatus') according to a first embodiment of the present invention.
Fig. 2 is a block diagram showing inner configuration of the steam sterilization apparatus of Fig. 1.
Fig. 3 is an exploded perspective view showing an injection gun, a heating amplifying means and a cleaning nozzle employed in the steam sterilization apparatus of Fig. 1.
Fig. 4 is a sectional view showing the heating amplifying means provide at an end of the injection gun employed in the steam sterilization apparatus of Fig. 1.
Fig. 5 is an exploded perspective view showing the heating amplifying means employed in the steam sterilization apparatus of Fig. 1.
Fig. 6 is a sectional view showing another example of an amplification tube employed in the steam sterilization apparatus of Fig. 1.
Fig. 7 is a perspective view showing one example of a delaying means provided in the amplification tube employed in the steam sterilization apparatus of Fig. 1.
Figs. 8(a) to 8(d) are sectional views taken along the lines a-a', b-b', c-c' and d-d' of Fig. 7, respectively.
Fig. 9 is a perspective view showing another example of the amplification tube employed in the steam sterilization apparatus of Fig. 1.
Fig. 10 is a sectional view taken along the line A-A of Fig. 9.
Fig. 11 is a sectional view showing that the cleaning nozzle is coupled to an end of the heating amplifying means of the steam sterilization apparatus of Fig. 1.
Fig. 12 is a sectional view showing a heating amplifying means employed in a steam sterilization apparatus according to a second embodiment of the present invention.
Fig. 13 is a sectional view showing a heating amplifying means employed in a steam sterilization apparatus according to a third embodiment of the present invention.
Fig. 14 is a sectional view showing a heating amplifying means employed in a steam sterilization apparatus according to a fourth embodiment of the present invention.
Fig. 15 is a perspective view showing an amplification tube and a delaying means employed in the heating amplifying means of Fig. 14.
Fig. 16 is a sectional view showing a heating amplifying means employed in a steam sterilization apparatus according to a fifth embodiment of the present invention.

### < Reference Symbols of Essential Parts >

100: steam generator 200: injection gun
300: flow tube
400, 600, 700, 800: heating amplifying means
500: cleaning nozzle

### BEST MODE

Hereinafter, preferred embodiments of the present invention will be described in detail with reference to the accompanying drawings. Prior to the description, it should be understood that the terms used in the specification and the appended claims should not be construed as limited to general and dictionary meanings, but interpreted based on the meanings and concepts corresponding to technical aspects of the present invention on the basis of the principle that the inventor is allowed to define terms appropriately for the best explanation. Therefore, the description proposed herein is just a preferable example for the purpose of illustrations only, not intended to limit the scope of the invention, so it should be understood that other equivalents and modifications could be made thereto without departing from the spirit and scope of the invention.

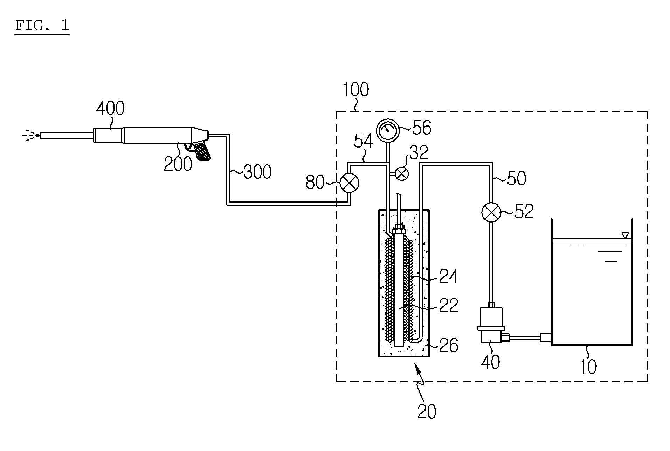

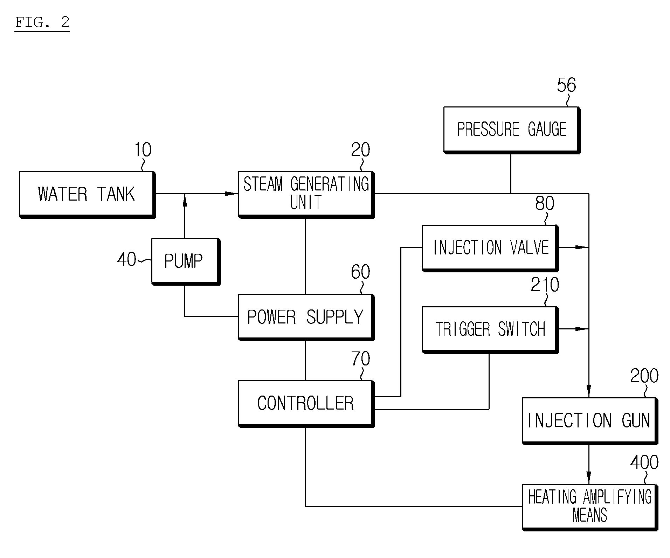

Fig. 1 shows main components of an apparatus for sterilization by steam (hereinafter, referred to as a steam sterilization apparatus) according to a first embodiment of the present invention, based on their functions, and Fig. 2 is a block diagram showing each component.

Referring to Figs. 1 and 2, the steam sterilization apparatus of the first embodiment includes a steam generator 100 for instantly heating water to generate a steam, an injection gun 200 for injecting the steam generated by the steam generator 100, a heating amplifying means 400 installed at the injection gun 200 to reheat the steam, and a flow tube 300 installed to connect the steam generator 100 to the injection gun 200.

The steam generator 100 includes a water tank 10 configured to store a predetermined amount of water, and a steam generating unit 20 for instantly heating the water supplied from the water tank 10 to generate a steam.

The water tank 10 is filled with water through a water supply hole (not shown), and the water stored in the water tank 10 is supplied to the steam generating unit 20 through a water supply tube 50 by means of a driving force of a pump 40. Preferably, a one-directional check valve 52 is installed at the water supply tube 50 so as to prevent backflow of water.

The steam generating unit 20 includes a heating bar 22 heated with a power source supplied from a power source 60 connected to an external power terminal, a winding water tube 24 wound around an outer periphery of the heating bar 22, and an insulation material 26 surrounding an outer side of the winding water tube 24.

The winding water tube 24 is successively connected to the water supply tube 50 extending from the water tank 10 and heats and evaporates the supplied water to generate a steam. For this purpose, the winding water tube 24 is preferably made of metal containing copper or its alloy with excellent thermal conductivity. More preferably, the winding water tube 24 is wound to configure subsequent laminates from the outside to the inside on the outer periphery of the heating bar 22, so that the water flowing therein is gradually heated from the outside and entirely evaporated at the inner side.

At this time, the power supplied to the heating bar 22 may be adjusted in order to control a temperature of the steam generated by the steam generating unit 20. For example, a greater power than a common one is supplied to the heating bar 22 in order to obtain a steam of a higher temperature, and a smaller power than a common one is supplied to the heating bar 22 so as to obtain a steam of a lower temperature. The supply of power may be controlled by a controller 70.

The steam generated at the winding water tube 24 is transferred to the injection gun 200 through the steam supply tube 54 and the flow tube 300.

Reference symbol 56 in Fig. 1 represents a pressure gauge. The pressure gauge 56 measures a pressure of steam on a flow path in real time. When a measured pressure is higher than a predetermined value, the pressure gauge 56 sends its signal to the controller 70. The controller 70 receiving the signal may control a safety valve 32 to be operated such that the steam is partially discharged outwards.

The flow tube 300 gives a path along which the steam generated by the steam generating unit 20 moves to the injection gun 200, and the flow tube 300 is generally made of a flexible hose made of synthetic resin or plastic. More preferably, a non-woven fabric or other insulation material may surround an outer part of the flow tube 300 such that a heat loss is not generated during the steam flowing process.

Though the steam generator 100 of the present invention has been illustrated based on the figures, the present invention is not limited thereto, but any steam generator may be adopted in the present invention if it can heat water and generate a steam.

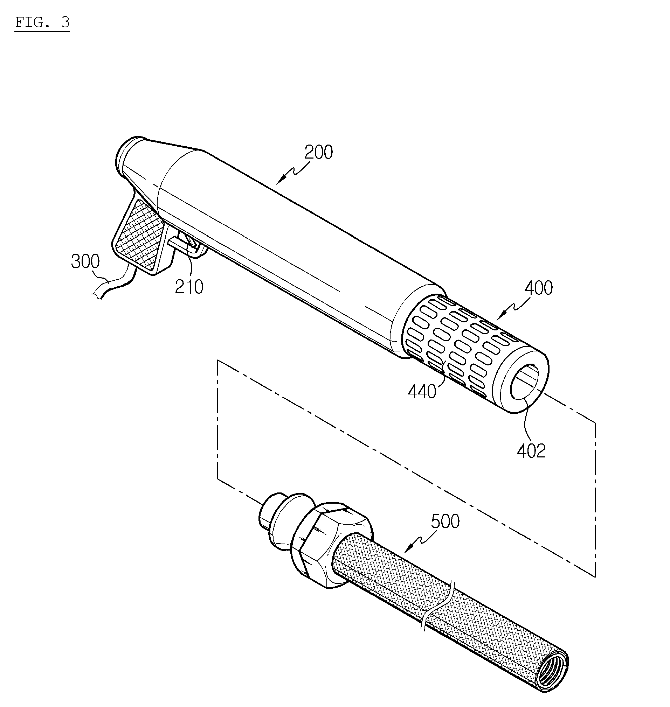

The injection gun 200 is used for finally injecting the steam, as schematically shown in Fig. 3.

As shown in Fig. 3, the injection gun 200 is connected to the flow tube 300 and receives the steam generated by the steam generator 100. The injection gun 200 may intermittently inject a steam by manipulation of a trigger switch 210.

The trigger switch 210 is connected to the controller 70 and its manipulation signal to the controller 70. The controller 70 opens or closes an injection valve 80 in accordance with the manipulation signal to inject a steam through the injection gun 200 or intercept injection of the steam.

According to the first embodiment of the present invention, the heating amplifying means 400 is provided at an end of the injection gun 200 so that the steam moved through the flow tube 300 is reheated to a predetermined temperature before being injected.

As necessary, a coupling part 402 for attaching or detaching a cleaning nozzle 500 may be prepared at an end of the heating amplifying means 400.

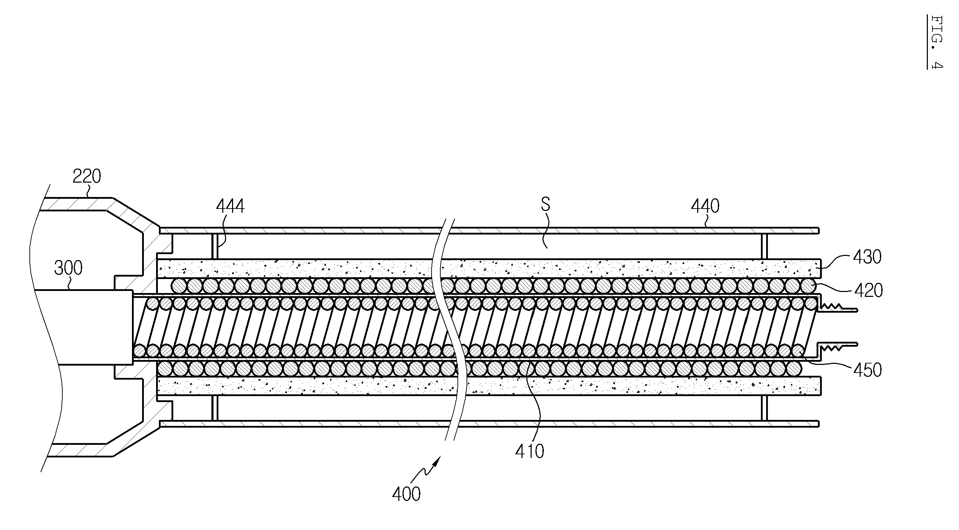

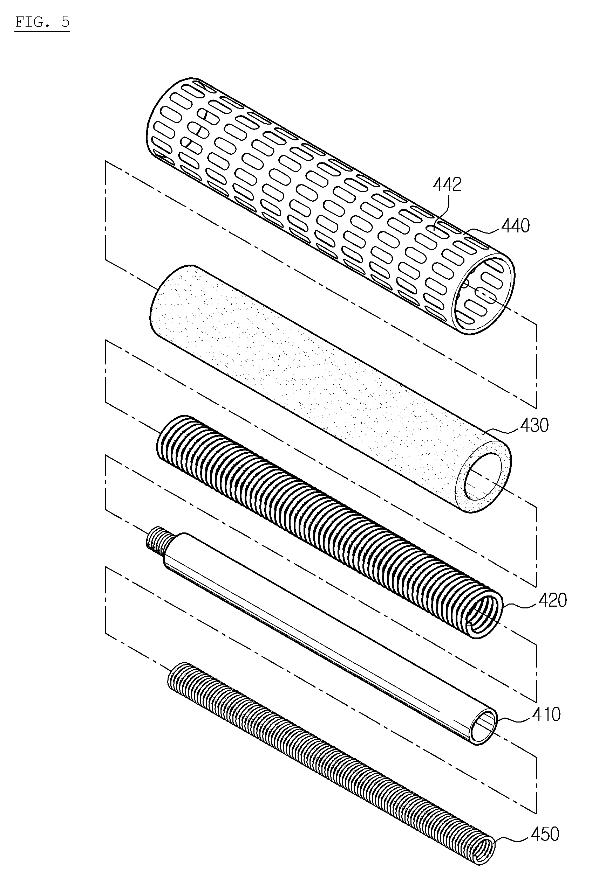

Figs. 4 and 5 show the heating amplifying means 400 in more detail. Referring Figs. 4 and 5 together, it would be understood that the heating amplifying means 400 is coupled to an end of a housing 220 of the injection gun 200. Though not shown in detail in the drawings, the heating amplifying means 400 may be detachably coupled to the end of the housing 220 of the injection gun 200 by means of screwing or fitting. As an alternative, the heating amplifying means 400 may be integrally coupled to the end of the housing 220 of the injection gun 200 during a production process.

The heating amplifying means 400 according to the present invention includes an amplification tube 410 connected to an end of the flow tube 300 located in the housing 220 of the injection 200, and the amplification tube 410 allows a stem to flow therein.

The amplification tube 410 is made of metal material, preferably stainless steel. The amplification tube 410 allows a steam to be finally heated and injected, and its size and length may be suitably set in accordance with its usage.

A heating means for heating the amplification tube 410 by a power applied from the power source 60 is provided at an outer periphery of the amplification tube 410. In this embodiment, the heating means is a heating coil 420 wound around the outer periphery of the amplification tube 410. However, the heating means of the present invention is not limited to the heating coil 420, and various kinds of heating means capable of heating the amplification tube 410 may be adopted.

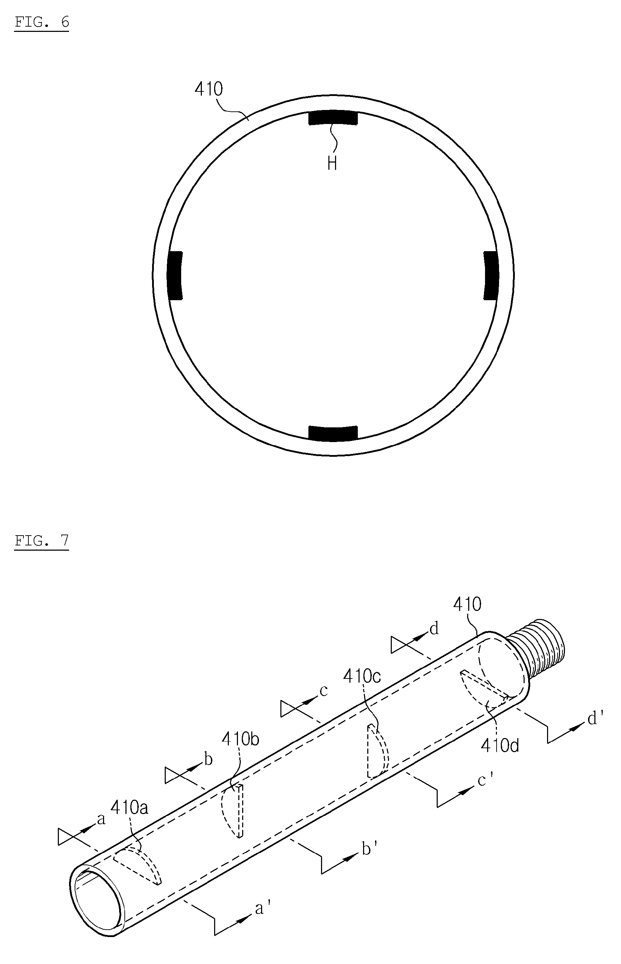

As another example of the heating means, it is possible to install a heat plate H at a part of an inner side of the amplification tube 410 as shown in Fig. 6 instead of the heating coil 420, and it is also possible to install the heat plate H together with the heating coil 420.

The heating means is preferably configured to control its temperature by means of the controller 70. If the temperature of the heating means is adjusted, it is possible to control temperature and pressure of the steam. For example, if a steam with higher temperature is demanded, the controller 70 supplies more power to the heating coil 420 and/or the heat plate H to increase temperature of the heating coil 420 and/or the heat plate H. On the contrary, if a steam with lower temperature is demanded, the controller 70 supplies less power to the heating coil 420 and/or the heat plate H to lower temperature of the heating coil 420 and/or the heat plate H.

Meanwhile, an insulation layer 430 is provided at an outer side of the heating coil 420 to prevent the heat from being lost outwards. Preferably, a support tube made of stainless steel may be separately provided to surround and support the insulation layer 430.

A heat sink 440 is provided at an outermost side of the heating amplifying means 400, and the heat sink 440 plays a role of preventing a user from being burned and protecting an inner configuration by lowering a surface temperature of the heating amplifying means 400.

For this purpose, a plurality of circulation holes 442 are formed in the heat sink 440, and a spacer 444 is protruded on an inner side of the heat sink 440 such that the heat sink 440 is spaced from the insulation layer 4430 by a predetermined interval. Thus, an external air may be freely circulated to an inner space S through the circulation holes 442, so the surface temperature of the heat sink 440 may be lowered to a suitable level.

Preferably, the heating amplifying means 400 may further include a delaying means for lowering a flow rate of the steam flowing in the amplification tube 410 such that the steam can be heated to the maximum. For example, a spring 450 inserted into the amplification tube 410 may be used as the delaying means. However, the delaying means is not limited to the spring, and any configuration having a shape similar to the spring 450 or functioning to delay a flow of the steam may be used as the delaying means. Examples of the delaying means are shown in Figs 7 to 10.

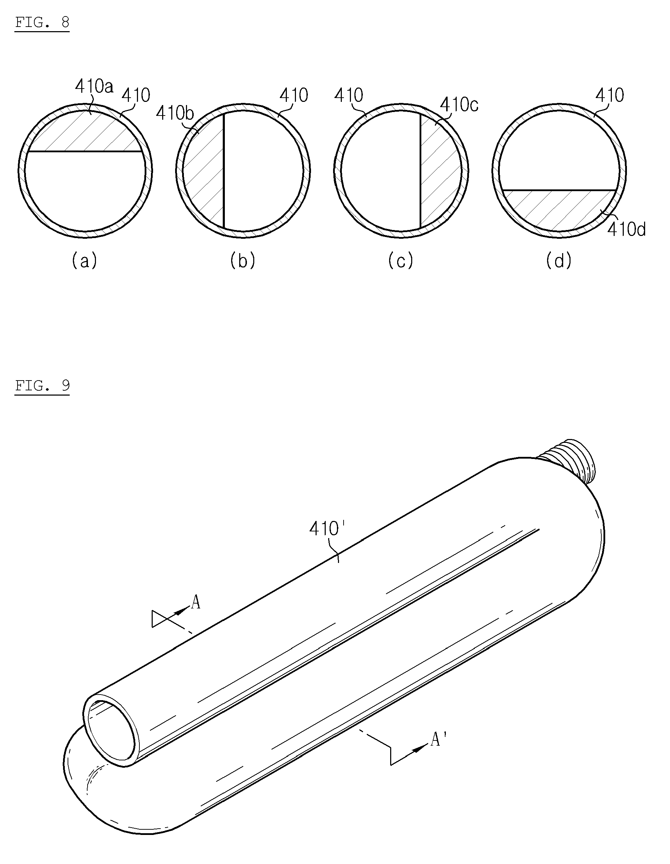

As shown in Figs. 7 and 8, a plurality of interference plates 410a, 410b, 410c, 410d are subsequently formed at an inner side of the amplification tube 410 such that the flowing steam may be collided with the interference plates to lower its flow rate. More preferably, the interference plates 410a, 410b, 410c, 410d may be designed to alternate at different locations so that the collision of steam is further promoted.

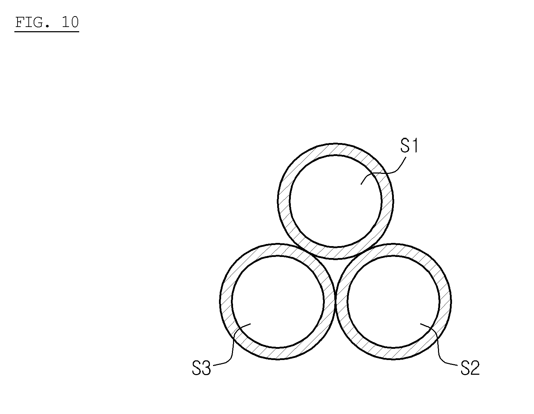

Meanwhile, the delaying means may be configured by bending an amplification tube 410' to form three passages S1, S2, S3, as shown in Figs. 9 and 10. In this case, the steam introduced into an inlet of the amplification tube 410' passes the passages S1, S2, S3 in order, thereby ensuring sufficient heating as much as the delay time.

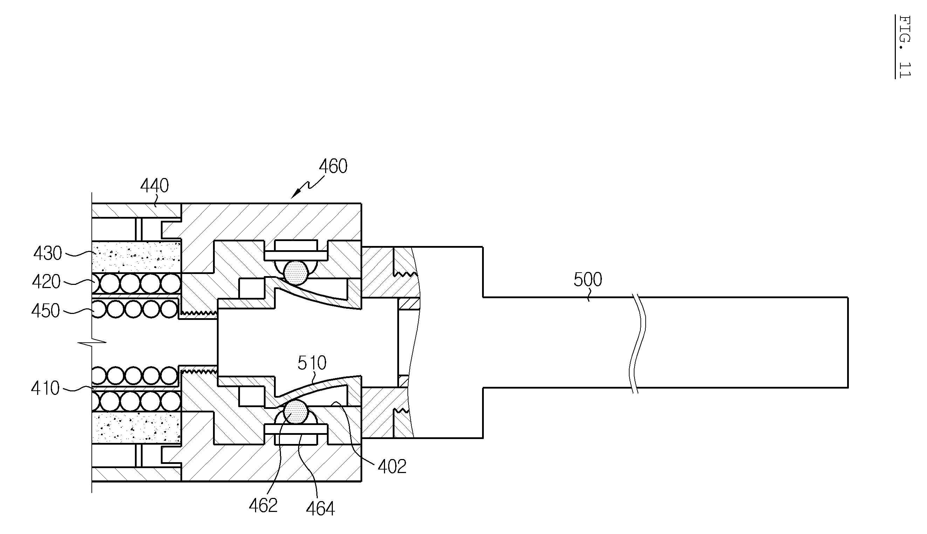

According to the present invention, an outlet of the amplification tube 410 may have a suitable coupling means for coupling with the cleaning nozzle 500, mentioned above, as exemplarily shown in Fig. 11.

Referring to Fig. 11, a coupler 460 for attaching or detaching the cleaning nozzle 500 is provided at the outlet of the heating amplifying means 400.

The coupler 460 may be fixed to the end of the amplification tube 410 by means of screwing, but the coupler 460 may be connected to the end of the amplification tube in various ways, not limited to the above.

At an inner side of the coupling part 402 of the coupler 460, at least one elastic locking ball 462 is installed. The elastic locking ball 462 is elastically supported by an elastic member 464.

Thus, if an oviduct-shaped coupling member 510 formed at a coupling end of the cleaning nozzle 500 is inserted into the coupling part 402 of the coupler 460, the elastic locking ball 462 retreats due to an elastic force of the elastic member 464 and then fastens again, thereby coupling the cleaning nozzle 500 to the coupler 460.

However, this coupling method is just one example, and the method of coupling the cleaning nozzle 500 to the heating amplifying means 400 according to the present invention can be modified in various ways.

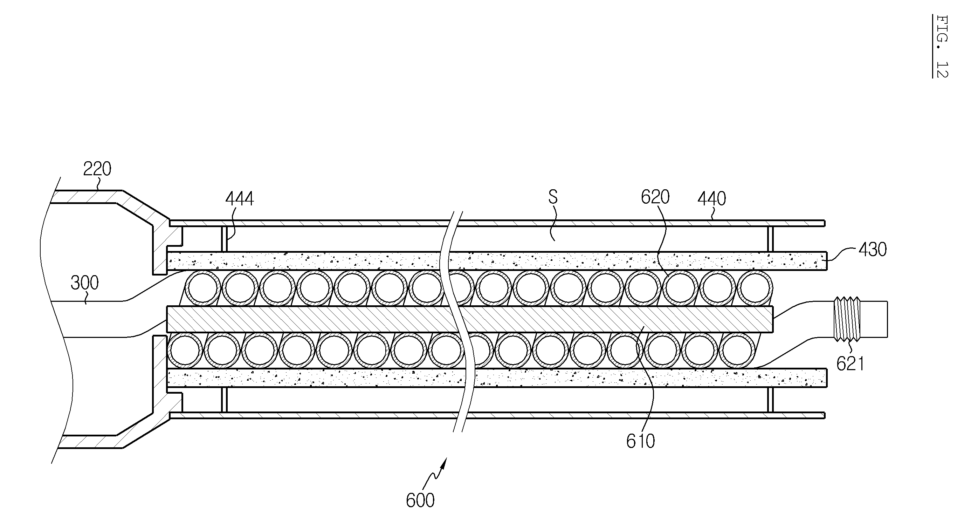

Meanwhile, the heating amplifying means of the steam sterilization apparatus according to the present invention may have various structures, as shown in Figs. 12 to 16. In Figs. 12 to 16, the same reference symbol as in Figs. 1 to 11 represents the substantially same component.

Meanwhile, the steam sterilization apparatus shown in Figs. 12 to 16 includes a steam generator, an injection gun for injecting the steam generated by the steam generator, a heating amplifying means installed at the injection gun to reheat the steam, and a flow tube installed to connect the steam generator to the injection gun. Among the above components, the steam generator, the injection gun and the flow tube are respectively identical to the steam generator 100, the injection gun 200 and the flow tube 300 of the first embodiment.

The heating amplifying means is most preferably installed at a front end of the injection gun, but it may also be installed at a middle or rear end of the injection gun. It would be easily understood by a person of ordinary skill in the art, who refers to this specification.

Fig. 12 is a sectional view showing a heating amplifying means employed in a steam sterilization apparatus according to a second embodiment of the present invention.

The heating amplifying means 600 includes a heater 610, a steam moving tube 620 wound around the heater 610, and an insulation layer 430 installed at an outer side of the wound steam moving tube 620.

The heater 610 may be heated by an external power and/or a power supplied from the power source 60, and the heater 610 has a rod shape.

The steam moving tube 620 is wound around the rod-shaped heater 610, and the steam moving tube 620 is installed in communication with the flow tube 300. Thus, the steam having moved through the flow tube 300 is heated by the heater 610 while moving in the steam moving tube 620, and then the steam is discharged to the cleaning nozzle 500 (not shown in Fig. 12). Reference symbol 621 represents a thread for coupling with the coupling part 402.

The insulation layer 430 is substantially identical to the insulation layer 430 of the first embodiment, and the insulation layer 430 is installed at the outer side of the steam moving tube 620 to intercept the heat transferred from the heater 610 and/or the steam moving tube 620 to the outside.

The heat sink 440 is preferably installed at the outside of the insulation layer 430. The heat sink 440 is substantially identical to the heat sink 440 of the first embodiment, so it is not explained in detail here.

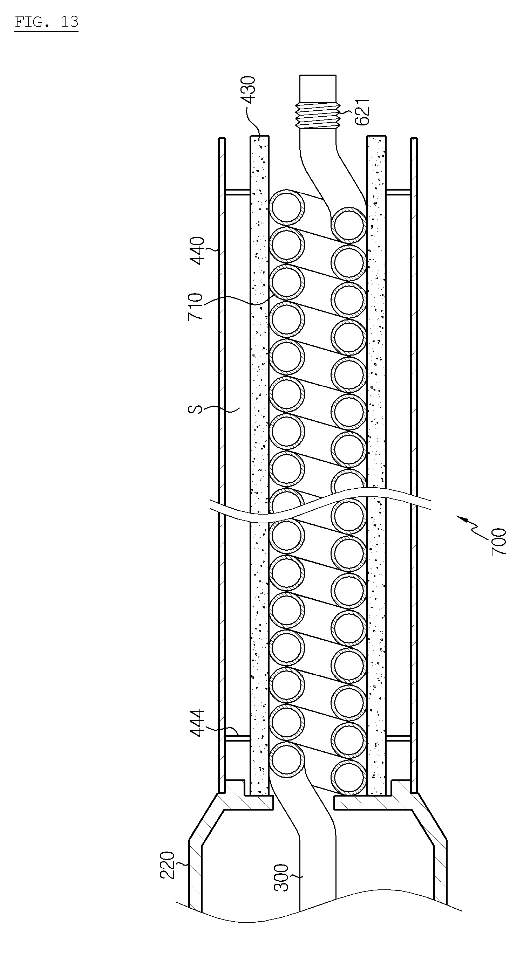

Fig. 13 is a sectional view showing a heating amplifying means employed in a steam sterilization apparatus according to a third embodiment of the present invention.

The heating amplifying means 700 includes a heating coil tube 710 and an insulation layer 430.

The heating coil tube 710 is wound into a coil shape, and the heating coil tube 710 is installed in the insulation layer 430. The heating coil tube 710 is communicated with an end of the flow tube 300, and the heating coil tube 710 may be heated by an external power and/or a power supplied from the power source 60. Thus, the steam having moved through the flow tube 300 is heated while moving in the heating coil tube 710. The heated steam is discharged to the cleaning nozzle 500 (not shown in Fig. 13).

The insulation layer 430 is substantially identical to the insulation layer 430 of the first embodiment, and the insulation layer 430 is installed at the outer side of the heating coil tube 710 to intercept the heat transferred from the heating coil tube 710 to the outside.

The heat sink 440 is preferably installed at the outside of the insulation layer 430. The heat sink 440 is substantially identical to the heat sink 440 of the first embodiment, so it is not explained in detail here.

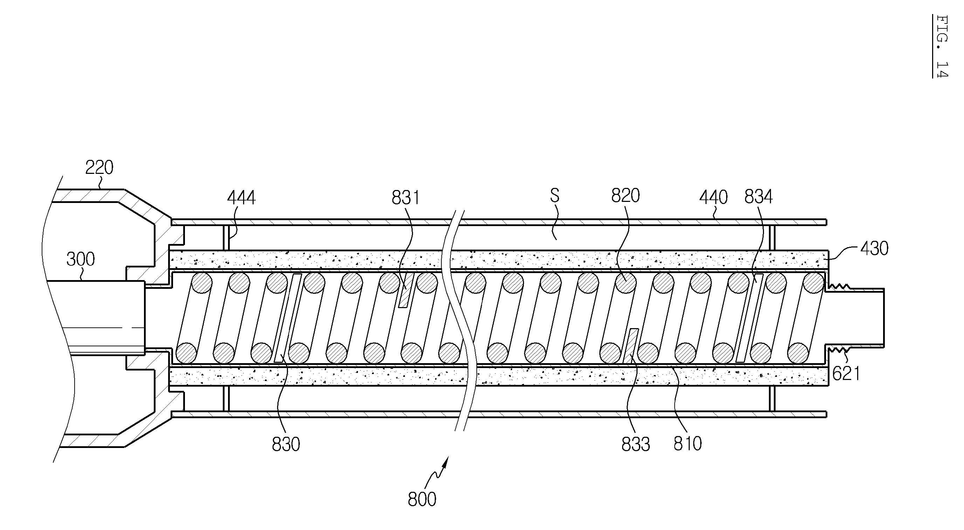

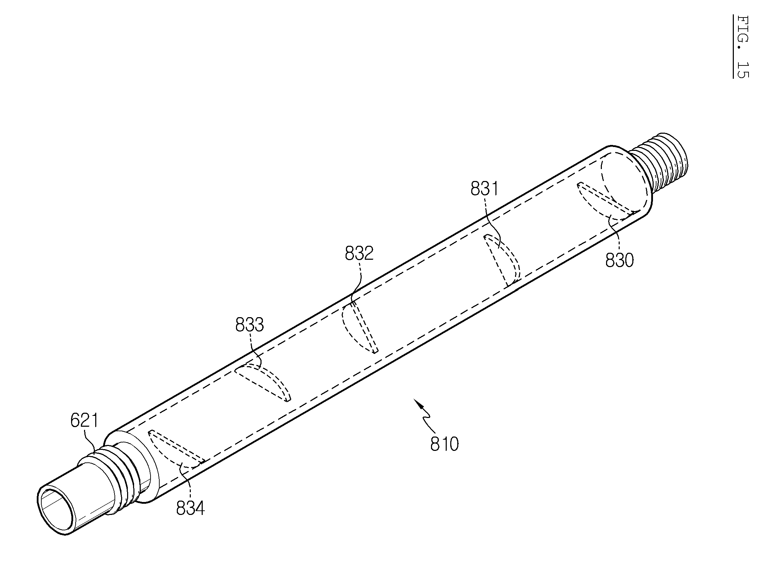

Meanwhile, Fig. 14 is a sectional view showing a heating amplifying means employed in a steam sterilization apparatus according to a fourth embodiment of the present invention, and Fig. 15 is a perspective view showing an amplification tube and a delaying means, which are employed in the heating amplifying means.

The heating amplifying means 800 includes an amplification tube 810, a heating coil 820 installed in the amplification tube 810, a delaying means for delaying a moving speed of the steam moving through the amplification tube 810, and an insulation layer 430 installed around the amplification tube 810.

The amplification tube 810 is communicated with an end of the flow tube 300. Thus, the steam having moved through the flow tube 300 moves in the amplification tube 810.

The heating coil 820 is wound into a coil shape, and the heating coil 820 is heated by an external power and/or a power supplied from the power source 60. Since the heating coil 820 is installed in the amplification tube 810, the steam moving in the amplification tube 810 is heated.

The delaying means lowers a moving speed of the steam so that the steam may be heated to a higher temperature. The delaying means may have various structures. Preferably, the delaying means is a plurality of interference plates 830, 831, 832, 833, 834 alternately formed at different locations of the inner side of the amplification tube 810.

The insulation layer 430 is substantially identical to the insulation layer 430 of the first embodiment, and the insulation layer 430 is installed at the outer side of the amplification tube 810 to intercept the heat transferred from the amplification tube 810 to the outside.

The heat sink 440 is preferably installed at the outside of the insulation layer 430. The heat sink 440 is substantially identical to the heat sink 440 of the first embodiment, so it is not explained in detail here.

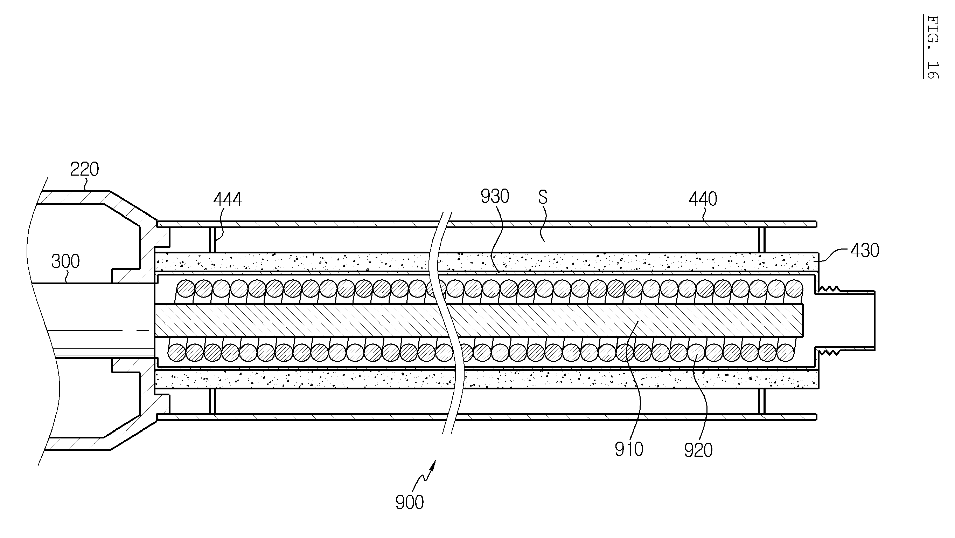

Fig. 16 is a sectional view showing a heating amplifying means employed in a steam sterilization apparatus according to a fifth embodiment of the present invention.

The heating amplifying means 900 includes an amplification tube 930, a heater 910 installed in the amplification tube 930, a coil 920 wound around the heater 910, and an insulation layer 430 installed around the amplification tube 930.

The amplification tube 930 is connected to an end of the flow tube 300, and the steam having moved through the flow tube 300 moves therein.

The heater 910 may be heated by an external power and/or a power supplied from the power source 60. The heater has a rod shape elongated in a length direction, and its section preferably has a circular shape. In case the heater 910 has a circular section, the coil 920 may be easily wound, and also it is easy to keep a gap between the coil 920 and the heater 910 constantly.

The coil 920 is wound around the heater 910 along a length direction of the heater 910. The coil 920 lowers a moving speed of the steam so that the steam may be heated to a higher temperature.

Preferably, the coil 920 is wound so as to be spaced apart from the heater 910 and the amplification tube 930 by predetermined intervals, respectively. Thus, the steam may move between the coil 920 and the heater 910 and between the coil 920 and the amplification tube 930.

Meanwhile, the coil 920 may play a role of heating the steam. In other words, it is possible to supply power to the coil 920 to heat the coil 920 such that the steam is heated by the heater 910 and the coil 920.

The insulation layer 430 is substantially identical to the insulation layer 430 of the first embodiment, and the insulation layer 430 is installed at the outer side of the amplification tube 930 to intercept the heat transferred from the amplification tube 930 to the outside.

The heat sink 440 is preferably installed at the outside of the insulation layer 430. The heat sink 440 is substantially identical to the heat sink 440 of the first embodiment, so it is not explained in detail here.

Now, operations of the steam sterilization apparatus according to the first embodiment of the present invention will be explained. Operations of the steam sterilization apparatus according to the second, third and fourth embodiments may be easily understood from the following explanation by a person of ordinary skill in the art, so they are not explained in detail here.

A user may use the steam sterilization apparatus in order to clean piping of a chilling/heating machine or a water purifier or interior of an air conditioner.

If power is supplied to the steam sterilization apparatus according to the present invention such that the steam sterilization apparatus is in an operation state, water stored in the water tank 10 flows to the steam generating unit 20 along the water supply tube 50 by means of a driving force of the pump 40.

The water flowing as mentioned above passes through the winding water tube 24 connected to the water supply tube 50, and in this process the water circulates on an outer periphery of the heating bar 22 heated to a high temperature such that the water is evaporated and converted into steam.

Subsequently, the generated steam is transferred to the injection gun 200 via the flow tube 300.

A user may put the cleaning nozzle 500 to a corresponding spot of the chilling/heating machine, the water purifier or the air conditioner to be cleaned, and then inject the steam by manipulating the trigger switch 210 of the injection gun 200.

In other words, if the user pulls the trigger switch 210, its signal is sent to the controller 70, and the controller 70 opens the injection valve 80 to inject the steam from the injection gun 200 through the flow tube 300.

In this process, the steam reaching the injection gun 200 via the flow tube 300 is reheated by the heating amplifying means 400 before being finally injected.

Though not shown in the figures, it is preferred to prepare an injection temperature control switch at the injection gun 200 or the steam generating unit 20 so that a user may set a steam temperature as desired within a predetermined range, for example about 100 to 250°C. If the user sets the injection temperature control switch to a predetermined temperature, the controller 70 controls an amount of power supplied to the heating bar 22 to adjust a temperature of the heating bar 22, thereby controlling a temperature of the steam. As an alternative or along with the above, it is also possible that, if a user sets the injection temperature control switch to a predetermined temperature, the controller 70 controls an amount of power supplied to the heating coil 420 to adjust a temperature of the steam.

Once the steam comes out of the flow tube 300 of the injection gun 200 and flows into the amplification tube 410 of the heating amplifying means 400, the steam is heated to a predetermined temperature by the heating coil 420 surrounding the amplification tube 410 and is then discharged and injected through the cleaning nozzle 500.

In this process, the spring 450 serving as a delaying means installed in the amplification tube 410 lowers a flowing speed of the steam such that the steam may be heated to a sufficiently high temperature.

### INDUSTRIAL APPLICABILITY

The steam sterilization apparatus according to the present invention reheats the steam, generated by the steam generator, by means of the heating amplifying means at the end of the injection gun just before being finally injected, to increase a temperature of the steam, thereby ensuring excellent effects in removing bacteria or molds or cleaning.

In addition, since the steam sterilization apparatus according to the present invention reheats a steam already generated, its heating effect is excellent and also it is possible to obtain a steam with a temperature suitable for its usage within a range of about 100 to 250°C by means of temperature control.

## Claims

1. An apparatus for sterilization by steam, comprising:
a steam generator for heating water to generate a steam;
an injection gun for injecting the steam generated by the steam generator;
a flow tube acting as a path through which the steam generated by the steam generator moves to the injection gun; and
a heating amplifying means installed at the injection gun to reheat the steam, moved through the flow tube, to a predetermined temperature before being injected.

2. The apparatus for sterilization by steam according to claim 1, wherein the heating amplifying means includes:
an amplification tube through which the steam moved via the flow tube is moved;
a heating means for heating the amplification tube; and
an insulation layer for preventing a heat of the heating means from transferring to the outside.

3. The apparatus for sterilization by steam according to claim 1, wherein the heating amplifying means includes:
a heater with a rod shape;
a steam moving tube wound around the heater so as to be capable of being heated by the heater, the steam moving tube allowing the steam to move therein; and
an insulation layer installed to an outer side of the steam moving tube wound around the heater,
wherein the steam moved via the flow tube is heated by the heater while the steam is moving through the steam moving tube.

4. The apparatus for sterilization by steam according to claim 1, wherein the heating amplifying means includes:
a heating coil tube installed in connection with the flow tube and allowing the steam to be movable therein, the heating coil tube being wound into a coil shape and capable of being heated by a power source; and
an insulation layer installed to an outer side of the heating coil tube wound into a coil shape,
wherein the steam moved via the flow tube is heated while the steam is moving through the heating coil tube.

5. The apparatus for sterilization by steam according to claim 1, wherein the heating amplifying means includes:
an amplification tube through which the steam moved via the flow tube moves;
a heating coil installed in the amplification tube and capable of being heated by a power source, the heating coil being wound into a coil shape;
a delaying means installed in the amplification tube to delay a moving speed of the steam that is moving through the amplification tube; and
an insulation layer installed around the amplification tube,
wherein the steam moving through the amplification tube is heated by the heating coil.

6. The apparatus for sterilization by steam according to claim 1, wherein the heating amplifying means includes:
an amplification tube through which the steam moved via the flow tube moves;
a heater (910) installed in the amplification tube and capable of being heated by a power source, the heater having a rod shape;
a coil (920) wound around the heater along a length direction of the heater in the amplification tube; and
an insulation layer installed around the amplification tube,
wherein the steam moving through the amplification tube is heated by the heater (910).

7. The apparatus for sterilization by steam according to claim 6,
wherein the heater (910) and the coil (920) are installed at a predetermined interval such that the steam is capable of moving between the heater (910) and the coil (920), and the amplification tube and the coil (920) are installed at a predetermined interval such that the steam is capable of moving between the heating amplification tube and the coil (920).

8. The apparatus for sterilization by steam according to claim 2, further comprising a delaying means for decreasing a flow rate of the steam moving in the amplification tube.

9. The apparatus for sterilization by steam according to claim 8,
wherein the delaying means include a spring inserted into the amplification tube.

10. The apparatus for sterilization by steam according to claim 5 or 8,
wherein the delaying means include a plurality of interference plates (410a)(410b)(410c)(410d) alternately formed at different locations of an inner side of the amplification tube.

11. The apparatus for sterilization by steam according to claim 2,
wherein the amplification tube has a bent shape to form a plurality of passages (S1)(S2)(S3).

12. The apparatus for sterilization by steam according to claim 2, further comprising a heat plate (H) additionally installed to a part of an inner side of the amplification tube.

13. The apparatus for sterilization by steam according to claim 2,
wherein a coupler (460) for attaching or detaching a cleaning nozzle (500) is installed at an outlet of the heating amplifying means.

14. The apparatus for sterilization by steam according to any one of claims 2 to 9, further comprising a heat sink (440) provided at an outside of the insulation layer, the heat sink having a plurality of circulation holes (442) formed therein and a spacer (444) protruded on an inner side thereof.
